# EUROPÄISCHE PATENTANMELDUNG

(11) **EP 0 547 377 A2**
(43) Veröffentlichungstag der Anmeldung: **23.06.1993**
(21) Anmeldenummer: 92119430.4
(22) Anmeldetag: 13.11.1992
(51) Int. Cl.: G01T 1/166, G01T 1/29, A61B 6/00

(54) **Stativ für einen Röntgenapparat**

(30) Priorität: 20.11.1991 CH 3394/91
(71) Anmelder: Schraner, Urs, CH-5342 Neuenhof (CH)
(72) Erfinder: Schraner, Urs, CH-5342 Neuenhof (CH)
(74) Vertreter: Fillinger, Peter, Dr.

(57) **Zusammenfassung**

Das Stativ hat eine raumfest vertikal montierbare Säule (1). An der Säule (1) ist eine Befestigungsvorrichtung (2) gelagert, welche in der Längsrichtung der Säule (1) verschiebbar und in der Längsebene der Säule (1) drehbar ist. An der Befestigungsvorrichtung (2) ist ein Querträger (3) angeordnet, der beidseitig der Befestigungsvorrichtung (2) je einen Arm (4,5) aufweist. Der eine Arm (4) dient der Aufnahme einer Röntgenröhre (14) mit einer Lichtvisiervorrichtung (15); der andere Arm (5) dient der Aufnahme einer Röntgenkassette (17). Um den Abstand der Röntgenröhre (14) und der Röntgenkassette (17 von der Befestigungsvorrichtung (2) längs des Querträgers (3) einzustellen, sind die beiden Arme (4,5) des Querträgers (3) in der Befestigungsvorrichtung (2) teleskopisch gelagert. Dadurch wird der Platzbedarf des Stativs in jeder Richtung verhältnismässig gering, und die Handhabung des Stativs wird erleichtert.

## Beschreibung

Die vorliegende Erfindung bezieht sich auf ein Stativ für einen eine Röntgenröhre und eine Röntgenkassettte enthaltenden Röntgenapparat gemäss dem Oberbegriff des Patentanspruchs 1.

Bei bekannten, im Handel erhältlichen und in diesbezüglichen Publikationen dargestellten und beschriebenen Stativen ist an der an der Säule vertikal verschiebbaren und in einer Vertikalebene drehbaren Befestigungsvorrichtung ein einteiliger Querträger fest angebracht, welcher sich mit je einem Arm koaxial auf beide Seiten der Befestigungsvorrichtung erstreckt. Auf dem einen Arm wird die Röntgenröhre und auf dem anderen Arm die Röntgenkassette angebracht. Um in der erforderlichen Weise den gegenseitigen Abstand von Röntgenröhre und Röntgenkassette verändern zu können, sind die Röntgenröhre und die Röntgenkassette auf ihren Armen in Längsrichtung des Querträgers verschiebbar angeordnet, wobei die Verschiebung manuell oder motorisch sowie individuell oder gekuppelt erfolgen kann.

Die bekannten Stative weisen den Nachteil auf, dass seitlich der vertikal montierten Säule in jeder Richtung, also sowohl horizontal als auch vertikal, im Behandlungszimmer ein freier Raum vorhanden sein muss, welcher durch die Gesamtlänge des an der Säule angebrachten Querträgers für die Röntgenröhre und die Röntgenkassette bestimmt ist. Diese Bedingung erschwert das Einrichten des Röntgenapparats bei gedrängten Platzverhältnissen und erschwert zudem wegen der grossen Länge des Querträgersdessen Handhabung für die Positionierbedingungen bei den üblichen, stark unterschiedlichen Aufnahmeverfahren.

Es ist Aufgabe der vorliegenden Erfindung, ein Stativ der eingangs genannten Art zu schaffen, dessen Platzbedarf in jeder Richtung verhältnismässig gering und dessen Handhabung erleichtert ist.

Zur Lösung dieser Aufgabe weist das erfindungsgemässe Stativ das im kennzeichnenden Teil des Patentanspruchs 1 angeführten Merkmal auf.

Da beim erfindungsgemässen Stativ die Länge des Querträgers durch den erforderlichen Abstand zwischen der Röntgenröhre und der Röntgenkassette bestimmt ist, nicht aber durch eine grössere Länge eines alle Einstellerfordernisse von Röntgenröhre und Röntgenkassette berücksichtigenden Querträgers, wird durch die Erfindung eine kompakte Bauweise mit verringertem Platzbedarf erzielt. Da ferner kein Querträger fester Länge vorliegt, ist die Handhabung des Stativs beim Einrichten bezüglich der gewünschten Aufnahme wesentlich erleichtert. Zudem erlaubt das erfindungsgemässe Stativ ohne weiteres Aufnahmen bzw. Untersuchungen auf der Hinter- oder Vorderseite des Patienten, indem die Röntgenkassette entweder unter bzw. über einer Patientenliege positioniert wird.

Gemäss Patentanspruch 2 können die beiden teleskopisch verschiebbaren Arme über eine Antriebsvorrichtung mechanisch miteinander gekuppelt sein, derart, dass die Arme gleichzeitig in entgegengesetzten Richtungen verschiebbar sind. Dadurch wird die Einstellung der Röntgenröhre und der Röntgenkassette bezüglich des zu untersuchenden Patienten erleichtert.

Die Antriebsvorrichtung kann hierbei für jeden Arm ein zugeordnetes Antriebselement aufweisen, wobei das Uebersetzungsverhältnis zwischen dem einen Antriebselement und dem zugeordneten Arm verschieden vom Uebersetzungsverhältnis zwischen dem anderen Antriebselement und dem zugeordneten Arm ist. Dadurch wird erreicht, dass die Drehmoment des die Röntgenröhre tragenden Arms und des die Röntgenkassette tragenden Arms im an der Säule befindlichen Drehpunkt mindestens angenähert entgegengesetzt gleich gross sind, so dass bei jeder eingestellten Distanz zwischen der Röntgenröhre und der Röntgenkassette und bei jeder Lage des drehbaren Querträgers mindestens angenähert Gleichgewicht bezüglich des Drehpunkts vorliegt.

Weitere vorteilhafte Ausführungsformen des Erfindungsgegenstandes sind in den nachfolgenden Patentansprüchen definiert.

Ausführungsbeispiele des erfindungsgemässen Stativs werden nachstehend anhand der Zeichnungen erläutert. Es zeigen:
Fig 1 und 2 eine Vorder- und eine Seitenansicht des Stativs mit Röntgenröhre und Röntgenkassette bei horizontal eingestelltem Querträger;
Fig. 3 und 4 eine Vorder- und eine Draufsicht der die Arme des Querträgers führenden Befestigungsvorrichtung mit angedeuteten Antriebselementen für die teilweise dargestellten Arme;
Fig. 5 einen Horizontalschnitt durch die Drehachse der an der Säule mit vertikaler Lage des Querträgers gemäss Fig. 2 angebrachten Befestigungsvorrichtung und
Fig. 6 eine Vorderansicht wie Fig. 1 eines zweiten Ausführungsbeispiels und
Fig. 7 und 8 eine Vorder- und eine Draufsicht des Querträgers wie in Fig. 3, wobei Teile weggebrochen bzw. weggelassen sind.

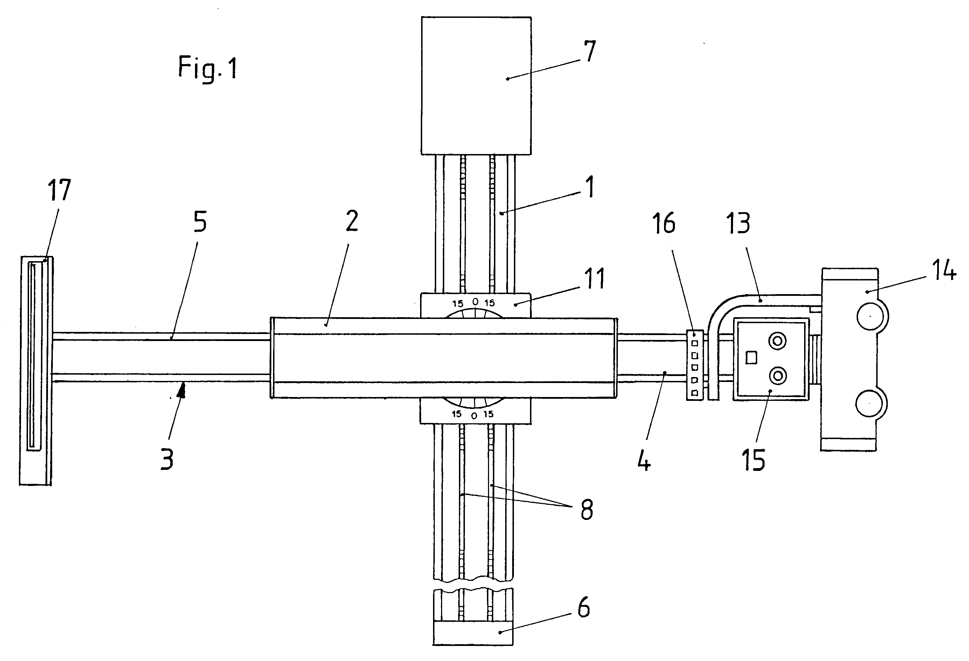

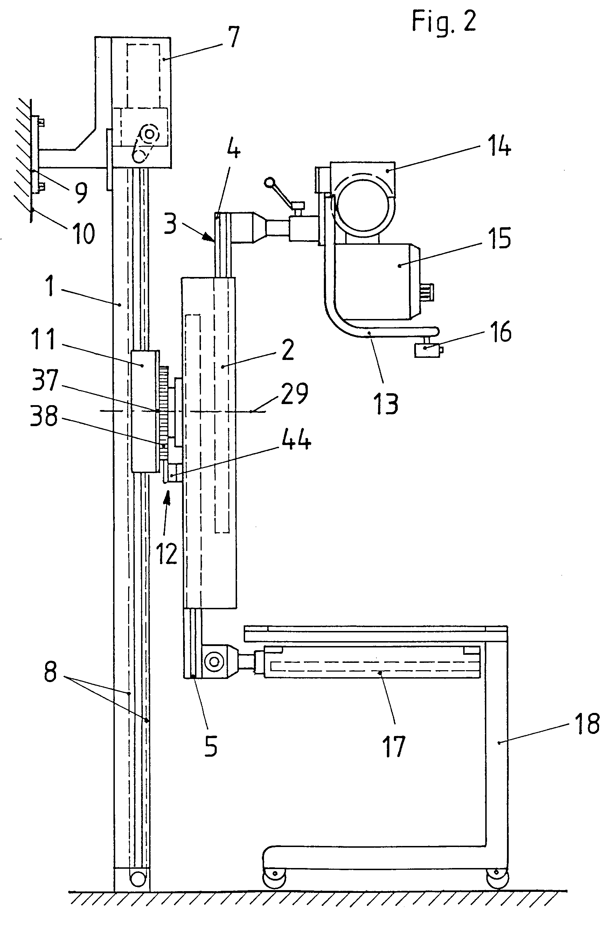

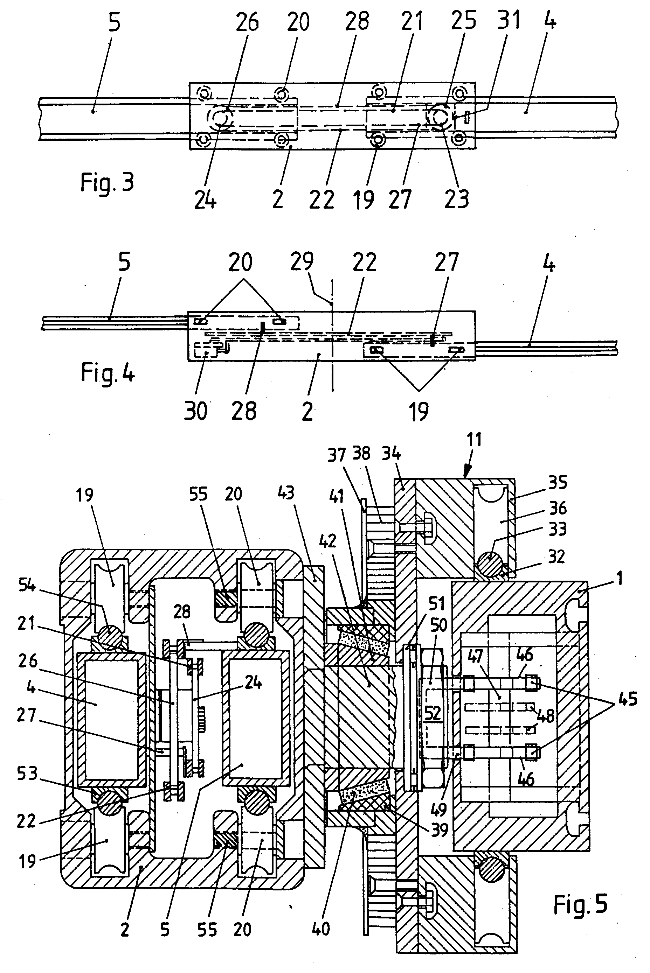

Gemäss Fig. 1 und 2 umfasst das vorliegende Stativ eine vertikale Säule 1, eine mit der Säule 1 verbundene Befestigungsvorrichtung 2 und einen allgemein mit 3 bezeichneten Querträger 3, in welchem zwei beidseitig der Befestigungsvorrichtung 2 herausragende, teleskopartig verschiebbare Arme 4 und 5 gelagert sind. Wie ersichtlich bestehen die Säule 1, die Befestigungsvorrichtung 2 und die Arme 4, 5 im wesentlichen aus kastenförmigen Profilstücken, wie dies nachstehend anhand der Fig. 5 noch näher erläutert wird.

Die Säule 1 ist an ihrem unteren Ende mit einem Sockel 6 (Fig. 1) und an ihrem oberen Ende mit einer Antriebseinheit 7 (Fig. 1, 2) versehen. Die Antriebseinheit 7 dient der gesteuerten Bewegung zweier in Längsnuten der Säule 1 befindlichen endlosen Ketten 8. Im Sockel 6 befindet sich eine nicht näher dargestellte Umlenkvorrichtung für die Ketten 8. Die Antriebseinheit 7 ist mit einer Platte 9 versehen, welche die in Fig. 2 dargestellte Befestigung der Säule 1 in vertikaler Position an einer Wand 10 erlaubt. Auch der Sockel 6 kann in nicht dargestellter Weise an der Wand 10 befestigt sein.

An den beiden parallelen Ketten 8 ist eine Dreheinheit 11 befestigt. Diese trägt über einen nachfolgend anhand von Fig. 5 noch näher beschriebenen Drehmechanismus 12 die Befestigungsvorrichtung 2 derart, dass die letztere gegenüber der Säule 1 in an sich bekannter Weise um bis zu 360° in einer zur Längsrichtung der Säule 1 parallelen Ebene schwenkbar ist.

In der Befestigungsvorrichtung 2 sind die beiden teleskopartig verschiebbaren Arme 4 und 5 vertikal in gleicher Höhe und horizontal nebeneinander parallel zueinander geführt. Am äusseren Ende des Arms 4 sind über einen um 360° schwengbaren Bügelarm 13 eine Röntgenröhre 14, eine Lichtvisiervorrichtung 15 und ein mit Drucktasten versehener Bedienungsteil 16 für das Stativ befestigt. Am äusseren Ende des anderen Arms 5 ist eine Röntgenkassette 17 (auch "Bucky" genannt) um 360° drehbar angebracht. In Fig. 2 ist zudem eine Patientenliege 18 dargestellt, unter welcher die Röntgenkassette 17 positioniert werden kann. Es ist ersichtlich, dass zufolge der teleskopartigen Lagerung der beiden Arme 4 und 5 in der Befestigungsvorrichtung 2 die Längs- ausdehnung des Trägers 3 jeweils nur den benötigten Abstand der Röntgenröhre 14 von der Röntgenkassette 17 beansprucht, das heisst, dass keine über die Röntgenröhre 14 und die Röntgenkassette in Längsrichtung vorstehenden, störenden Abschnitte des Trägers 3 vorhanden sind. Das Fehlen solcher vorstehender Trägerabschnitte erlaubt es auch, problemlos Untersuchungen auf der Oberseite eines auf der Patientenliege 18 befindlichen Patienten vorzunehmen.

Jeder Arm 4, 5 kann an sich individuell und manuell in der Befestigungsvorrichtung 2 verschoben werden, um einen zweckmässigen Abstand der Röntgenröhre 14 von der Röntgenkassette bzw. der Röntgenröhre 14 und der Röntgenkassette 17 von der Drehachse 29 der Befestigungsvorrichtung 2 zu erzielen. Dies ist ohne Kraftaufwand wegen der nachstehend noch im einzelnen erläuterten, leichtgängigen, aber spielfreien Lagerung der Arme 4, 5 in der Befestigungsvorrichtung ohne grossen Kraftaufwand möglich. Da jedoch die individuelle Einstellung der beiden Armlängen verhältnismässig zeitraubend wäre und anhand von Längsskalen vorgenommen werden müsste, ist es von Vorteil, die Längsbewegungen der beiden Arme 4, 5 miteinander mechanisch zu kuppeln. Ein diesbezügliches Ausführungsbeispiel ist in den Fig. 3 und 4 schematisch dargestellt.

Wie ersichtlich, sind die beiden Arme 4 und 5 im Innern der Befestigungsvorrichtung 2 je auf vier paarweise angeordneten Rollen 19 bzw. 20 gelagert. Zwischen den beiden Armen 4, 5 sind zwei endlose Ketten 21 bzw. 22 angeordnet, die in den Endbereichen der Befestigungsvorrichtung 2 über Kettenräder 23, 24 bzw. 25, 26 geführt sind. Der Arm 4 ist mit der Kette 21 über einen Mitnehmer 27 und der Arm 5 mit der Kette 22 über einen Mitnehmer 28 fest verbunden. Ferner ist mindestens eines der auf der gleichen Achse angeordneten Kettenrad-Paare 23, 24 bzw. 25, 26 drehfest ausgebildet, so dass bei der Bewegung der einen Kette auch die andere Kette mitbewegt wird. Beispielsweise sind die linksseitigen Kettenräder 24, 26 fest auf ihrer Welle montiert und miteinander verschraubt, während die rechtsseitigen Kettenräder 23, 25 frei laufend auf ihrer Welle gelagert sind. Da der eine Arm 4 über den Mitnehmer 27 mit dem oberen Trum der zugeordneten Kette 21 verbunden ist, der andere Arm 5 über den Mitnehmer 28 dagegen mit dem unteren Trum der zugeordneten Kette 22, erfolgt die gekuppelte Bewegung der beiden Arme 4, 5 gegenläufig.

Aus Fig. 3 ist ferner ersichtlich, dass die Kettenräder 23, 24 des Antriebs des Armes 4 einen kleineren Durchmesser als die Kettenräder 25, 26 des Armes 5 haben. Dies hat zur Folge, dass bei einer Drehbewegung der Kettenräder der Arm 4, welcher die Röntgenröhre 14 trägt, langsamer läuft als der die Röntgenkassette 17 tragende Arm 5. Dadurch kann erreicht werden, dass sich die an der Drehachse 29 (Fig. 4) wirksamen, durch die Röntgenröhre 14 und gegensinnig durch die Röntgenkassette 17 ausgeübten Drehmomente kompensieren, also ein Gleichgewicht zwischen Röntgenröhre 14 und Röntgenkassette 17 besteht, und zwar für jede Distanz zwischen diesen beiden und für jede Schräglage des Querträgers 3. Falls das angestrebte Gleichgewicht nach der Montage der Röntgenröhre 14 und der Röntgenkassette 17 nicht vorhanden ist, kann der Gleichgewichtszustand durch einmaliges Verschieben der Röntgenröhre 14 oder der Röntgenkassette auf den sie tragen Armen 4 bzw. 5 leicht erzielt werden.

Die gekuppelte Einstellung der beiden Arme 4, 5 kann rein manuell durch Einschieben bzw. Ausziehen eines der beiden Arme erfolgen. Eine andere, ebenfalls manuelle Einstellmöglichkeit kann darin bestehen, die das Kettenradpaar 23, 25 oder 24, 26 tragende Welle auf der Aussenseite der Befestigungsvorrichtung 2 mit einem Drehrad oder Drehgriff zu versehen. Schliesslich ist es auch möglich, einen motorischen Antrieb für die genannten Kettenräder vorzusehen und über eine der Drucktasten des Bedienungsteils 16 (Fig. 1, 2) zu steuern.

Um zu verhindern, dass sich die eingestellte Lage der leichtgängigen Arme 4, 5 bei einer Schräglage des Querträgers 3 verschiebt, ist gemäss Fig. 4 eine auf die eine Kette 21 wirksame Distanz-Arretiervorrichtung 30 vorgesehen, die beispielsweise durch Knopfdruck betätigt bzw. gelöst werden kann. Ferner ist es vorteilhaft, das eine Paar der Kettenräder 23, 25 bzw. 24, 26 mit einem in Fig. 3 angedeuteten Kettenspanner 31 zu versehen.

Anhand der Fig. 5 wird im folgenden ein beispielsweiser Aufbau der Vertikal-Positionierung und der Drehpositionierung des Trägers 3 sowie der teilweise bereits anhand der Fig. 3 und 4 erläuterten Verschiebung der beiden Arme 4 und 5 des Trägers 3 beschrieben.

Die Säule 1 besteht aus einem Hohlkasten-Profilstück, längs welchem die als Wagen ausgebildete Dreheinheit 11 verschiebbar ist, vergleiche auch Fig. 1 und 2. Als Führung für die Dreheinheit 11 sind an den beiden Schmalseiten der Säule 1 je eine Wellenklemmschiene 32 angebracht, in welche eine geschliffene Welle 33 eingepresst ist. Die Dreheinheit 11 hat eine Grundplatte 34, an welcher zwei Führungsstücke 35 angeschraubt sind. Jedes Führungsstück 35 enthält Rollen 36, die auf den Wellen 33 laufen. Auf der Vorderseite der Grundplatte 34 ist zudem ein Ring 37 aufgeschraubt,der mit einer Aussenverzahnung 38 versehen ist. Ferner trägt die Grundplatte 34 einen Aussenring 39 eines Kegellagers, dessen mit Rollen 40 versehener Innenring 41 auf einem Bolzen 42 fest angebracht ist, welcher in einen Flanschring 43 eingepresst ist. Der Flanschring 43 ist an der Rückwand der Befestigungsvorrichtung 2 angebracht, welche ebenfalls ein kastenförmiges Profil hat. Die Befestigungsvorrichtung 2 ist demnach gegenüber der Grundplatte 34 der Dreheinheit 11 um 360° drehbar. Um die Befestigungseinheit 2 in einer gewählten Drehlage festzuhalten, ist an der Befestigungsvorrichtung 2 gemäss Fig. 2 eine Arretiervorrichtung 44 angebracht, die in die Aussenverzahnung 38 des Rings 37 eingreifen kann.

Für den Antrieb der Dreheinheit 11, das heisst zu ihrer Verschiebung längs der Säule 1 und damit zur entsprechenden Verschiebung der Befestigungsvorrichtung 2 ist im Innern der Säule 1 ein Kettenantrieb angeordnet. Dieser besteht u.a. aus den zwei parallelgeführten endlosen Ketten 8 , welche an den beiden Enden der Säule 1 über in Fig. 5 angedeutete Umlenk-Kettenräder 46 laufen. Auf der Welle 47 der oberen Umlenk-Kettenräder 46 sind zudem zwei weitere Kettenräder 48 angebracht, welche durch einen am oberen Ende der Säule 1 in der Antriebseinheit 7 (Fig. 1, 2) befindlichen Elektromotor angetrieben werden.

Die vorderen Trums der beiden Ketten 8 sind in Nuten 49 der Frontwand der Säule 1 positioniert und damit gegen aussen offen, wie dies bereits in Fig. 1 dargestellt ist. An den Ketten 8 ist ein Mitnehmerstück 50 befestigt, das ein U-förmiges Profil hat. Das Mitnehmerstück 50 ist mit der Grundplatte 34 und über ein Axial-Rillenkugellager 51 mit der Befestigungsvorrichtung 2 verbunden, wobei mit 52 ein Konterring bezeichnet ist.

Durch die Anwendung des beschriebenen Kettenantriebs lässt sich bezüglich der Länge der Säule 1 ein grosser Vertikalhub der Befestigungsvorrichtung 2 und damit des Querträgers 3 erzielen, beispielsweise ein Vertikalhub von 165 cm bei einer Säulenlänge von 185 cm. Damit kann der Vorteil der teleskopartig verschiebbaren Arme 4 und 5 voll ausgenutzt werden.

Zur Lagerung und Führung der beiden Arme 4 und 5 sind, wie anhand der Fig. 3 und 4 bereits angeführt, die Rollen 19 und 20 angeordnet. Aus Fig. 5 ist ersichtlich, dass die kastenförmigen Arme 4 und 5 an ihrer Ober- und Unterseite mit Klemmschienen 53 versehen sind, in welche geschliffene Wellen 54 eingepresst sind, die in den Rollen 19 bzw. 20 laufen. Die Rollen 19 und 20 sind mit zentrischen und vorzugsweise exzentrischen Bolzen 55 montiert, um eine spielfreie Lagerung der Arme 4 und 5 einstellen zu können. Die Rollen 19 und 20 können hierzu auch seitlich eingestellt werden.

In Fig. 5 sind auch zwei Kettenräder 24, 26 der insgesamt vier Kettenräder 23 bis 26 der Fig. 3 und 4 samt den durch sie geführten Ketten 21 und 22 dargestellt. Ferner sind aus Fig. 5 die Mitnehmer 27 und 28 für den Arm 4 bzw. 5 dargestellt. Hierbei ist, wie bereits anhand der Fig. 3 und 4 erwähnt, der die Röntgenröhre 14 tragende Arm 4 mit der über das kleinere Kettenrad 24 laufenden Kette 21 verbunden, während der die Röntgenkassette tragende Arm 5 mit der über das grössere Kettenrad 26 laufenden Kette 22 verbunden ist.

Da die Profilstücke der Säule 1, der Befestigungsvorrichtung 2 und der Arme 4, 5 aus Leichtmetall mit Vorteil aus Aluminium bestehen, ergibt sich ein sehr niedriges Gesamtgewicht des vorliegenden Stativs von beispielsweise 120 kg ohne die Röntgenröhre 14 und die Lichtvisiervorrichtung 15. Zudem lässt sich das vorliegende System modulweise in Baueinheiten aufteilen, die getrennt und zufolge der geringen Maximallänge von etwa 2 m in einem gewöhnlichen Kombiwagen bzw. in einem Personenlift transportiert und innert kurzer Zeit am Aufstellungsort zusammengesetzt, fertig montiert und angeschlossen werden können.

Die Bedienung des vorliegenden Stativs mit Röntgenröhre und Röntgenkassette ist sehr einfach, da alle Stativfunktionen am Bedienungsteil 16 (Fig. 1, 2) mit einer Hand bedient werden können. Die Lichtvisiervorrichtung 15, mit welcher ein Zentralstrahl auf den Mittelpunkt der Röntgenkassette 17 zentriert wird, ferner die Schwenkung der Befestigungsvorrichtung 2 bzw. des Trägers 3 der Arme 4, 5 und die Distanz-Arretiervorrichtung 30 sind einfach zu bedienen und können elektronisch gesteuert sein.

Zu erwähnen ist noch, dass das vorliegende Stativ auch als Hilfsgerät für EKG, Therapiegeräte usw. verwendet werden kann, da es möglich ist, die Patientenliege 18 (Fig. 2) unter das Ende des normalerweise die Röntgenkassette 17 tragenden Arms 5 zu fahren.

Beim beschriebenen Beispiel können der Querträger 3, die Röntgenkassette 17 und der Röntgenapparat 14 spiegelsymmetrisch ausgebildet bzw. angeordnet sein, so dass sich (bezogen auf Fig. 1) der Arm 4 mit der Röntgenröhre 14 links der Säule 1 und der Arm 5 mit der Röntgenkassette 17 rechts von der Säule 1 befinden. Damit kann die Säule 1 in einem kleinen Raum nach links näher gegen eine Wand gerückt werden, wogegen die Ausführungsform nach Fig. 1 (wegen des kleineren Schwenkradius des Armes 4 näher zu einer rechts stehenden Wand rückbar ist.

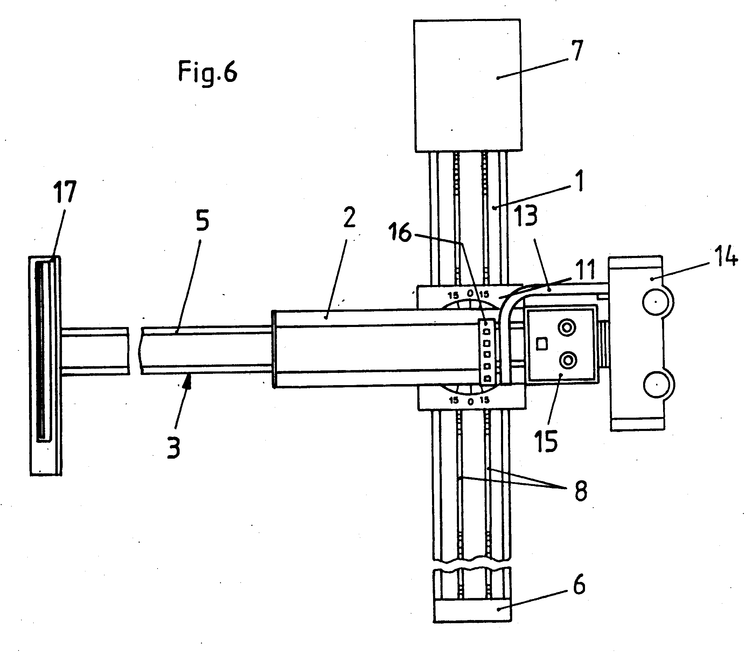

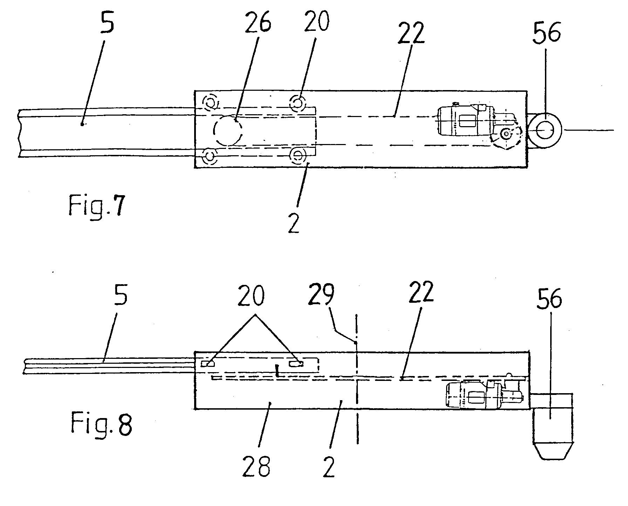

Bei noch engeren Platzverhältnissen kann, wie das Beispiel nach den Fig. 6 bis 8 zeigt, nur der die Röntgenkassette 17 tragende Arm 5 des Querträgers 3 längenverstellbar ausgebildet sein, wogegen die Röntgenröhre 14 mittels eines Adapters 56 an der Befestigungsvorrichtung 2 mit vorgegebenem Radialabstand zur Drehachse 29 angeordnet ist. Diese Ausführungsform hat zwar den Nachteil. dass nur bei einem bestimmten Auszug des Armes 5 ein Gleichgewichtszustand besteht in dem der Querträger 3 mühelos gedreht werden kann. Mit einer geeigneten Sicherheitsschaltung kann diese Gleichgewichtsauszugslage aber von einer Bedienungsperson problemlos eingestellt werden. Die Vorteile demgegenüber sind, dass in der Raumlänge - ohne Einschränkung für den Röntgenbetrieb - 50 bis 60 cm eingespart werden können, wobei auch der Raumbedarf für die Vertikalstellung kleiner wird.

## Patentansprüche

1. Stativ für einen eine Röntgenröhre (14) und eine Röntgenkassette (17) enthaltenden Röntgenapparat, mit einer raumfest vertikal montierbaren Säule (1) und mit einer an der Säule (1) gelagerten, in deren Längsrichtung verschiebbaren und in deren Längsebene um eine Drehachse (29) drehbaren Befestigungsvorrichtung (2) eines beidseitig der Drehachse der Befestigungsvorrichtung (2) wegragenden Querträgers (3) zur Aufnahme der Röntgenröhre (14) bzw. der Röntgenkassette (17), wobei Mittel (21 bis 28) vorhanden sind, um den Abstand der Röntgenröhre (14) und/oder der Röntgenkassette (17) von der Drehachse (29) der Befestigungsvorrichtung (2) längs des Querträgers (3) einzustellen, dadurch gekennzeichnet, dass der Querträger (3) mindestens einen längenverstellbaren Arm (4, 5) aufweist.

2. Stativ nach Anspruch 1, dadurch gekennzeichnet, dass der bzw. die längenverstellbaren Arme (4, 5) in der Befestigungsvorrichtung (2) teleskopisch verschiebbar gelagert und vorzugsweise mit einer Antriebsvorrichtung (21 bis 28 versehen ist bzw. sind.

3. Stativ nach Anspruch 2, dadurch gekennzeichnet, dass beide Arme (4,5) in der Befestigungsvorrichtung (2) teleskopisch verschiebbar gelagert ist.

4. Stativ nach Anspruch 2 od.3 dadurch gekennzeichnet, dass die beiden teleskopisch verschiebbaren Arme (4,5) zwei über die Antriebsvorrichtung (21 bis 28) mechanisch miteinander gekuppelte Einzelarme sind, derart, dass die Arme (4,5) gleichzeitig in entgegengesetzten Richtungen bewegbar sind.

5. Stativ nach Anspruch 3, dadurch gekennzeichnet, dass die Antriebsvorrichtung für jeden Arm (4; 5) ein zugeordnetes Antriebselement (21,23,24; 22,25,26) aufweist, wobei das Uebersetzungsverhältnis zwischen dem einen Antriebselement (21,23,24) und dem zugeordneten Arm (4) verschieden vom Uebersetzungsverhältnis zwischen dem anderen Antriebselement (22,25, 26) und dem zugeordneten Arm (5) ist.

6. Stativ nach einem der Ansprüche 2 bis 5, dadurch gekennzeichnet, dass die Antriebsvorrichtung für jeden Arm (4; 5) je eine endlose, über zwei Kettenräder (23,24; 25,26) geführte Kette (21; 22) und je einen die Kette (21; 22) mit dem Arm (4; 5) verbindenden Mitnehmer (27; 28) umfasst, wobei jeweils mindestens zwei der je einem der beiden Armen (4; 5) zugeordneten Kettenräder (23,25; 24,26) gleichachsig angeordnet und miteinander drehfest verbunden sind.

7. Stativ nach Anspruch 6, dadurch gekennzeichnet, dass das eine (23; 24) der gleichachsig angeordneten Kettenräder (23,25; 24,26) einen kleineren Durchmesser als das andere (25; 26) hat, derart, dass der eine Arm (4) sich bei einer Betätigung der Antriebsvorrichtung langsamer bewegt als der andere Arm (5).

8. Stativ nach einem der Ansprüche 4 bis 7, dadurch gekennzeichnet, dass der Antriebsvorrichtung (21 bis 28) eine Arretiervorrichtung (30) zugeordnet ist.

9. Stativ nach einem der Ansprüche 2 bis 8, dadurch gekennzeichnet, dass die Arme (4,5) in der Befestigungsvorrichtung (2) mittels Rollen (19,20) verschiebbar gelagert sind.

10. Stativ nach Anspruch 9, dadurch gekennzeichnet, dass die Rollen (19,20) in der Befestigungsvorrichtung (2) drehbar gelagert und die Arme (4,5) an gegenüberliegenden Seiten mit Längswellen (54) zur Aufnahme der Rollen (19,20) versehen sind.

11. Stativ nach einem der Ansprüche 3 bis 10, dadurch gekennzeichnet, dass die Arme (4,5) in der Befestigungsvorrichtung (2) in ihrer Längsrichtung parallel nebeneinander angeordnet sind.

12. Stativ nach einem der Ansprüche 1 bis 11, dadurch gekennzeichnet, dass zur Verschiebung der Befestigungsvorrichtung (2) längs der Säule (1) diese mit einem Kettenantrieb (8,46) versehen ist.

13. Stativ nach Anspruch 12, dadurch gekennzeichnet, dass der Kettenantrieb zwei parallel über an den Enden der Säule (1) angeordnete Umlenk-Kettenräder (46) geführte endlose Ketten (8) aufweist, mit deren vorderen, in Nuten (49) der Säule (1) liegenden Trums die Befestigungsvorrichtung (2) über einen längs der Säule (1) geführten Schlitten (11) und über Drehlager (39,40,41; 51) schwenkbar befestigt ist.

14. Stativ nach Anspruch 1, dadurch gekennzeichnet, dass die Röntgenröhre mit unveränderlichem Abstand mit Bezug auf die Drehachse (29) am Querträger (3) und die Röntgenkassette (17) am längenverstellbaren Arm (5) angeordnet ist.

15. Röntgenapparat, mit einer Röntgenröhre, einer Röntgenkassette und einem Stativ , welches eine raumfest vertikel montierbare Säule (1) und eine an der Säule (1) gelagerte, in deren Längsrichtung verschiebbare und in deren Längsebene drehbare Befestigungsvorrichtung (2) eines Querträgers (3) enthält, der beidseitig der Befestigungsvorrichtung (2) je einen Arm (4,5) aufweist, von welchen der eine Arm (4) die Röntgenröhre (14) und der andere Arm (5) die Röntgenkassette (17) in einem von der Drehachse der Befestigungsvorrichtung (2) einstellbaren Abstand trägt, dadurch gekennzeichnet, dass mindestens einer der beiden Arme (4,5) des Querträgers (3) längenverstellbar ist.

16. Röntgenapparat nach Anspruch 15, dadurch gekennzeichnet, dass mindestens einer der Arme (4,5) in der Befestigungsvorrichtung (2) teleskopisch verschiebbar gelagert ist.

17. Röntgenapparat nach Anspruch 16, dadurch gekennzeichnet, dass beide Arme (4,5) in der Befestigungsvorrichtung (2) teleskopisch verschiebbar gelagert ist.

18. Röntgenapparat nach Anspruch 17, dadurch gekennzeichnet, dass die beiden teleskopisch verschiebbaren Arme (4,5) zwei über eine Antriebsvorrichtung (21 bis 28) mechanisch miteinander gekuppelte Einzelarme sind, derart, dass die Arme (4,5) gleichzeitig in entgegengesetzten Richtungen bewegbar sind.

19. Röntgenapparat nach Anspruch 18, dadurch gekennzeichnet, dass das Uebersetzungsverhältnis der Antriebsvorrichtung (21 bis 28) bezüglich der beiden Arme (4,5) unterschiedlich ist, derart, dass sich bei einer Betätigung der Antriebsvorrichtung (21 bis 28) der die Röntgenröhre (14) tragende Arm (4) langsamer bewegt als der die Röntgenkassettte (17) tragende Arm (5).

20. Röntgenapparat nach einem der Ansprüche 17 bis 19, dadurch gekennzeichnet, dass die Arme (4,5) in ihrer Längsrichtung parallel nebeneinander angeordnet sind.
